# EUROPEAN PATENT SPECIFICATION

(11) **EP 1 632 222 B1**
(45) Date of publication and mention of the grant of the patent: **30.09.2009**
(21) Application number: 05004655.6
(22) Date of filing: 03.03.2005
(51) Int. Cl.: A61K 8/97, A61Q 5/00, A61Q 7/00

(54) **Hair restorer composition using oriental herbs**
Haarregenerierendes Mittel enthaltend orientale Kräuter
Composition régénérante capillaire comprenant des herbes orientales

(30) Priority: 27.07.2004 KR 2004058595
(43) Date of publication of application: 08.03.2006
(73) Proprietor: Lee, Byung-Su, Choongcheongnam-do 312-920 (KR)
(72) Inventor: Lee, Byung-Su, Choongcheongnam-do 312-920 (KR)
(74) Representative: Harrison Goddard Foote

(56) References cited:
- US-A1- 2003 152 545
- DATABASE WPI Section Ch, Week 200415 Derwent Publications Ltd., London, GB; Class B04, AN 2004-144559 XP002352870 & CN 1 453 011 A (XU S) 5 November 2003 (2003-11-05)

## Description

### BACKGROUND OF THE INVENTION

### 1. Field of the Invention

The present invention relates to a hair restorer composition using Oriental herbs, and more particularly, to a hair restorer composition using Oriental herbs, which can prevent loss of hair, suppress dandruff and itching, and improve a scalp, by periodically giving nutrition to a scalp, hair follicle, and hair.

### 2. Description of the Related Art

In general, shampoos or soaps are used to remove sebum, sweat, keratin, waste product, dust, etc., and rinses are used to give nutrition to a scalp and hair.

However, since the conventional shampoos or rinses primarily include chemical materials, skins may be stimulated. In addition, the shampoos or rinses cannot cure the itching well.

Furthermore, the shampoos or rinses do not have effects such as prevention of loss of hair and promotion of restoration of hair and may promote the loss of hair, to the contrary.

Therefore, there have been developed and studied various methods of producing a shampoo having a function of promoting restoration of hair and preventing loss of hair by using various Oriental herbs, which are helpful to human bodies and have been ever used.

### SUMMARY OF THE INVENTION

The present invention is contrived in view of the above-mentioned points. That is, the present invention is derived from the findings that an Oriental herb extract including ginseng and Chrysanthemum zawadskii has functions of promoting restoration of hair and preventing loss of hair, as results of repeated clinical tests for a long time using the extract which is obtained by combining various Oriental herbs in various ways and heating (boiling up the combined Oriental herbs with water.

The present invention provides a hair restorer composition using Oriental herbs, which can promote restoration of hair, prevent loss of hair, mitigate itching, give nutrition to hair, and improve a scalp.

According to an aspect of the present invention, there is provided a hair restorer composition using Oriental herbs, wherein the hair restorer composition is produced by mixing an Oriental herb extract of 10 to 40 wt%, a viscosity control agent of 0.1 to 15 wt%, a surfactant of 30 to 80 wt%, and an oil of 0.1 to 15 wt%, the Oriental herb extract being obtained by heating ginseng and Chrysanthemum zawadskii with water.

One or more additives selected from a group consisting of pigment, aromatic, and preservative may be further added by 0.01 to 10 wt% to the hair restorer composition according to the present invention.

In the hair restorer composition according to the present invention, the Oriental herb extract is obtained by adding Oriental herbs of 30 to 70 kg to water of 800 kg, preliminarily heating the mixture at 100°C for 3 to 8 hours, primarily heating the mixture for 48 hours or more so as to obtain an intermediate extract of 600 to 700 kg, and secondarily heating the intermediate extract so as to obtain a final extract of 400 to 500 kg.

The Oriental herbs of 30 to 70 kg include the ginseng of 5 to 30 kg and the Chrysanthemum zawadskii of 20 to 45 kg.

When the amount of each component used in the Oriental herbs is less than the lower limit, the component cannot provide a sufficient effect. On the other hand, when the amount of each component is more than the upper limit, the component provide an excessive effect, thereby causing a harmful effect.

When the Oriental herbs less than 30 kg are added to the water of 800 kg, a long heating time is required for obtaining the extract having a sufficient concentration. Therefore, energy may be greatly wasted, thereby increasing the production cost.

When the Oriental herbs more than 70 kg are added to the water of 800 kg, the concentration of the extract can be excessively increased. In addition, since the relative amount of water is small, necessary components cannot be sufficiently extracted from the Oriental herbs.

When the Oriental herbs of 30 kg are added to the water of 800 kg, it is preferable that the primary heating is performed until the intermediate extract is close to about 600 kg. When the Oriental herbs of 70 kg are added to the water of 800 kg, it is preferable that the primary heating is performed until the intermediate extract is close to about 700 kg. This is because the final extracts have a similar concentration.

When the Oriental herbs of 30 kg are added and the intermediate extract is close to 600 kg, it is preferable that the secondary heating is performed until the final extract is close to 400 kg. When the Oriental herbs of 70 kg are added and the intermediate extract is close to 700 kg, it is preferable that the secondary heating is performed until the final extract is close to 500 kg. This is because the final extracts have a similar concentration.

However, the present invention is not limited to the above-mentioned ranges, but the concentration of the extract may be adjusted as needed. The weight of the intermediate extract and the weight of the final extract may be also set within the aforementioned ranges, regardless of the amounts of the Oriental herbs.

Drinking water or distilled water is used as the water.

As known well, ginseng restores stamina, strengthens the lungs, improves the spleen (stomach), and secures the hearts. Ginseng is famous for providing the relief from fatigue, the enhancement of physical strength, the prevention of aging, the treatment and improvement of anemia, hypertension, and weakened heart, the release of stress, the treatment of neurosis, the improvement of autonomic ataxia, the treatment and improvement of diabetes, gastroenteric disorder, inappetency, diarrhea, constipation, tuberculosis, asthma, skin diseases, suppurative tumor, and psoriasis skin, and the smoothing of roughened skin.

It is known that ginseng contains 30 or more ginsenosides as major components and several kinds of phenol compounds and a maltol component as materials for the prevention of aging and the anti-fatigue.

It is also known that ginseng promotes the secretion of hormones relating to the release of stress to exhibit an anti-stress effect. Ginseng has adaptogenic activity to exhibit an effect of reinforcing the defensive force which can non-singularly resist various stimuli as causes of human diseases. In addition, ginseng improves learning ability and memory to enhance intellectual powers.

Furthermore, since ginseng includes manganese, copper, vanadium, cobalt, arsenic, germanium, phosphor, aluminum, nickel, etc. as microelements, ginseng can promote change of aged cells to new cells through germanium cell toxicity and has an anti-cancer effect.

It is known that Chrysanthemum zawadskii is a kind of Chrysanthemum to be effective for women diseases and keeping warm. Chrysanthemum zawadskii is also effective for headache, prevention of hair from loss, and prevention of hair from whitening. Chrysanthemum zawadskii is also effective for strengthening stomach, protecting stomach, stomach tonic, depurating blood, promoting appetite, apoplexy, neuralgia, etc.

The choline component contained in Chrysanthemum zawadskii is an important basic unit for constituting acetylcholine which is a chemical material relating to formation of memory in a brain, and it is known that the choline component plays an important role in forming a cell membrane of various tissues of a human body.

Since the hair restorer composition using Oriental herbs according to the present invention is produced by heating and extracting ginseng and zawadskii having the aforementioned functions and effects together with water, it is possible to promote restoration of hair, prevent loss of hair, remove dandruff, mitigate itching, give nutrition to scalp, hair follicle, and hair, and improve a scalp.

### DETAILED DESCRIPTION OF THE EMBODIMENTS

A hair restorer composition using Oriental herbs according to an exemplary embodiment of the present invention was produced, and ten adults were allowed to use the hair restorer composition. Then, the results have been examined.

### (Embodiment)

An intermediate extract of 650 kg was obtained by putting ginseng of 18 kg and Chrysanthemum zawadskii of 36 kg into a pot containing water of 800 kg, preliminarily heating the pot at 100°C for 5 hours, and primarily heating again the pot for 72 hours. A final extract of 450 kg was obtained by secondarily heating the intermediate extract of 650 kg. Then, the obtained Oriental herb extract (final extract) of 25 wt% was mixed with a viscosity control agent of 7 wt%, a surfactant of 60 wt%, a silicon oil of 7 wt%, a preservative of 0.2 wt%, and an aromatic of 0.8 wt%, thereby obtaining the hair restorer composition using Oriental herbs according to an embodiment of the present invention.

Ten adults were allowed to use the hair restorer composition two times per day (in the morning and night) for four months. In the meantime, results of observation of statuses of the scalp and hair are shown in Table 1.

**Table 1**

| | |
|---|---|
| First month | The itching of the scalp is mitigated |
| Second month | Soft hair is grown in the scalp |
| Third month | The grown hair gets black with its growth |
| Fourth month | The black hair is thickened |

Here, the statuses of the scalp and hair were observed with eyes. For the itching of the scalp, results of comparison with the normal statuses in which the embodiment of the present invention had not been used were represented.

As can be seen from Table 1, it was confirmed that most users had felt the mitigation of the itching during the first month.

As a result of using the hair restorer composition for two or more months, most users underwent that the soft hair is grown in the scalp, and the grown hair gets black and thicker with its growth.

Although not shown in Table 1, no user suffered from red spots or other skin stimuli.

In addition, although not shown in Table 1, the degree of refreshment due to use of the embodiment was examined, and it was confirmed as a result of the examination that the hair restorer composition according to the embodiment of the present invention allows the users to feel clean and fresh for a long time.

Table 2 shows the results of observing the statuses of the scalp and hair every month when five patients suffering from alopecia areata and five patients suffering from dandruff were allowed to use the hair restorer composition for four months.

**Table 2**

| | Patient suffering from alopecia areata | Patient suffering from dandruff |
|---|---|---|
| First month | No response | Dandruff is slightly decreased |
| Second month | No response | Dandruff is remarkably decreased |
| Third month | Growth of hair is increased around the hair-lost portion | No dandruff is generated |
| Fourth month | Soft hair is grown in a part of the hair-lost portion | No dandruff is generated |

In Table 2, the statuses of the scalp and hair were observed with eyes, similarly to in Table 1.

As shown in Table 2, the patients suffering from alopecia areata did not exhibit any particular response for the first two months, but some patients underwent the increasing growth of hair around the hair-lost portion for the third month. In addition, it could be seen with eyes that one patient underwent the growth of soft hair in the hair-lost portion for the fourth month.

Although not shown in Table 2, four patients did not undergo the growth of soft hair in the hair-lost portion, but underwent the increasing growth of hair around the hair-lost portion, by using the hair restorer composition according to the embodiment of the present invention for fourth months. As a result, hair got rich as a whole.

The patients suffering from dandruff underwent slight decrease of dandruff for the first month, but underwent remarkable decrease of dandruff for the second month. In addition, no dandruff was generated with use for three or more months. As a result, it could be seen that the generation of dandruff was completely cured.

Table 3 shows the results of observing the statuses of the scalp and hair every month when ten adult patients suffering from alopecia were allowed to use the hair restorer composition in every morning for four months and to use a generally-used shampoo (mainly made of chemical materials) in every night.

**Table 3**

| | |
|---|---|
| First month | The itching of scalp is mitigated |
| Second month | The itching of scalp is generated again |
| Third month | The restoration of hair and the status of scalp are not changed |
| Fourth month | The restoration of hair and the status of scalp are not changed |

In Table 3, the statuses of the scalp and hair were observed with eyes, similarly to in Table 1. For the itching of scalp, results of comparison with the normal statuses in which the embodiment of the present invention had not been used were represented.

From Table 3, it could be seen that the combined use of the hair restorer composition according to the embodiment of the present invention and the generally-used shampoo decreases the effect or exhibit no effect.

In case of the combined use, although not shown in Table 3, red spots which patients of alopecia can frequently have were caused for some patients.

In case of the patients with red spots, it could be confirmed that the red spots disappear and the itching is mitigated by stopping the combined use of the general shampoo and the hair restorer composition according to the embodiment of the present invention and using only the hair restorer composition two times in the morning and night every day (which is not shown in Table 3).

The hair restorer composition using Oriental herbs according to the embodiment of the present invention can decrease the harm to a human body and the environmental pollution, when it is compared with the generally-used shampoo mainly made of chemical materials.

When an extract is obtained by boiling down the Oriental herbs with water as described above (in a case of general Oriental prescription), the combination of various components provides a desired effect. Therefore, it is difficult to exactly find out the operating mechanism of a specific component. The Oriental prescription have verified and confirmed its effects on the basis of statistical numerical values from clinical tests for a long time.

Therefore, since it is difficult to sufficiently explain the operating mechanisms of the present invention, the effects of the present invention was checked through clinical tests on many patients.

Since the hair restorer composition using Oriental herbs according to the present invention described above requires only equipment for boiling down the Oriental herbs, it can be produced with simple equipment and with low cost.

The hair restorer composition using Oriental herbs according to the present invention can prevent loss of hair and promote restoration of hair. It can also periodically supply nutrition to scalp, hair follicle, and hair.

In addition, the hair restorer composition using Oriental herbs according to the present invention can mitigate dandruff and itching and improve the scalp.

Although the exemplary embodiments of the present invention have been described, the present invention is not limited to the exemplary embodiments, but may be modified in various forms without departing from the scope of the appended claims, and the detailed description, of the present invention. Therefore, it is natural that such modifications belong to the scope of the present invention.

## Claims

1. A hair restorer composition using Oriental herbs, wherein the hair restorer composition is produced by mixing an Oriental herb extract of 10 to 40 wt%, a viscosity control agent of 0.1 to 15 wt%, a surfactant of 30 to 80 wt%, and an oil of 0.1 to 15 wt% with each other, the Oriental herb extract being obtained by heating Oriental herbs with water,
wherein the Oriental herb extract is obtained by adding Oriental herbs of 30 to 70 kg to water of 800 kg, preliminarily heating the mixture at 100°C for 3 to 8 hours, primarily heating the mixture for 48 hours or more so as to obtain an intermediate extract of 600 to 700 kg, and secondarily heating the intermediate extract so as to obtain a final extract of 400 to 500 kg,
wherein the Oriental herbs of 30 to 70 kg include the ginseng of 5 to 30 kg and the Chrysanthemum zawadskii of 20 to 45 kg.

2. The hair restorer composition of claim 1, wherein one or more additives selected from a group consisting of pigment, aromatic, and preservative are further added by 0.01 to 10 wt% to the hair restorer composition.

3. Use of a composition as claimed in claim 1 or 2 in the manufacture of a composition for use in promoting restoration of hair and preventing loss of hair.

4. A method for the preparation of a hair restorer composition which comprises mixing an Oriental herb extract of 10 to 40 wt%, a viscosity control agent of 0.1 to 15 wt%, a surfactant of 30 to 80 wt%, and an oil of 0.1 to 15 wt% with each other, the Oriental herb extract being obtained by heating Oriental herbs with water,
wherein the Oriental herb extract is obtained by adding Oriental herbs of 30 to 70 kg to water of 800 kg, preliminarily heating the mixture at 100°C for 3 to 8 hours, primarily heating the mixture for 48 hours or more so as to obtain an intermediate extract of 600 to 700 kg, and secondarily heating the intermediate extract so as to obtain a final extract of 400 to 500 kg,
wherein the Oriental herbs of 30 to 70 kg include the ginseng of 5 to 30 kg and the Chrysanthemum zawadskii of 20 to 45 kg.

5. The method of claim 4, wherein one or more additives selected from a group consisting of pigment, aromatic, and preservative are further added by 0.01 to 10 wt% to the hair restorer composition.

## Patentansprüche

1. Haarregenerierendes Mittel enthaltend orientalische Kräuter, wobei das haarregenerierende Mittel durch Mischen von 10 bis 40 Gew.-% eines orientalischen Kräuterextrakts, 0,1 bis 15 Gew.-% eines viskositätsregelnden Agens, 30 bis 80 Gew.-% eines Tensids und 0,1 bis 15 Gew.-% eines Öls miteinander hergestellt wird, wobei das orientalische Kräuterextrakt durch Erhitzen orientalischer Kräuter mit Wasser erhalten wird,
wobei das orientalische Kräuterextrakt **dadurch** erhalten wird, dass 30 bis 70 kg orientalischer Kräuter 800 kg Wasser hinzugefügt werden, die Mischung bei 100°C für 3 bis 8 Stunden einer Vorerhitzung unterzogen wird, die Mischung für 48 Stunden oder mehr einer ersten Erhitzung unterzogen wird, um ein Zwischenextrakt von 600 bis 700 kg zu erhalten, und das Zwischenextrakt einer zweiten Erhitzung unterzogen wird, um ein Endextrakt von 400 bis 500 kg zu erhalten,
wobei die 30 bis 70 kg orientalischer Kräuter 5 bis 30 kg Ginseng und 20 bis 45 kg Chrysanthemum zawadskii enthalten.

2. Haarregenerierendes Mittel nach Anspruch 1, wobei zusätzlich ein oder mehrere Additive mit 0,01 bis 10 Gew.-% dem haarregenerierenden Mittel hinzugefügt werden, welche aus einer Gruppe ausgewählt werden, die aus Pigmenten, Aromaten und Konservierungsstoffen besteht.

3. Verwendung eines Mittels nach Anspruch 1 oder 2 bei der Herstellung eines Mittels zur Verwendung bei der Förderung der Regenerierung von Haar und der Vermeidung des Haarausfalls.

4. Verfahren zur Zubereitung eines haarregenerierenden Mittels, umfassend das Mischen von 10 bis 40 Gew.-% eines orientalischen Kräuterextrakts, 0,1 bis 15 Gew.-% eines viskositätsregelnden Agens, 30 bis 80 Gew.-% eines Tensids und 0,1 bis 15 Gew.-% eines Öls miteinander, wobei das orientalische Kräuterextrakt durch Erhitzen orientalischer Kräuter mit Wasser erhalten wird,
wobei das orientalische Kräuterextrakt **dadurch** erhalten wird, dass 30 bis 70 kg orientalischer Kräuter 800 kg Wasser hinzugefügt werden, die Mischung bei 100°C für 3 bis 8 Stunden einer Vorerhitzung unterzogen wird, die Mischung für 48 Stunden oder mehr einer ersten Erhitzung unterzogen wird, um ein Zwischenextrakt von 600 bis 700 kg zu erhalten, das Zwischenextrakt einer zweiten Erhitzung unterzogen wird, um 400 bis 500 kg eines Endextrakts zu erhalten,
wobei die 30 bis 70 kg orientalischer Kräuter 5 bis 30 kg Ginseng und 20 bis 45 kg Chrysanthemum zawadskii enthalten.

5. Verfahren nach Anspruch 4, wobei zusätzlich 0,01 bis 10 Gew.-% eines oder mehrerer Additive dem haarregenerierenden Mittel hinzugefügt werden, welche aus einer Gruppe ausgewählt werden, die aus Pigmenten, Aromaten und Konservierungsstoffen besteht.

## Revendications

1. Une composition capillaire régénérante utilisant des herbes orientales, où la composition capillaire régénérante est obtenue en mélangeant, les uns avec les autres, un extrait d'herbes orientales de 10 à 40 % en poids, un agent d'ajustement de viscosité de 0,1 à 15 % en poids, un agent tensio-actif de 30 à 80 % en poids, et une huile de 0,1 à 15 % en poids, l'extrait d'herbes orientales étant obtenu en chauffant des herbes orientales avec de l'eau,
où l'extrait d'herbes orientales est obtenu en ajoutant 30 à 70 kilogrammes d'herbes orientales à 800 kilogrammes d'eau, en chauffant tout d'abord le mélange à 100°C pendant 3 à 8 heures, puis pendant 48 heures
ou plus de façon à obtenir un extrait intermédiaire de 600 à 700 kilogrammes, et enfin en chauffant l'extrait intermédiaire de façon à obtenir un extrait final de 400 à 500 kilogrammes,
où les herbes orientales de 30 à 70 kilogrammes comprennent 5 à 30 kilogrammes de ginseng et 20 à 45 kilogrammes de chrysanthemum zawadskii.

2. La composition capillaire régénérante selon la revendication 1, où un ou plusieurs additifs choisis parmi un groupe se composant d'un colorant, d'un arôme, et d'un agent de conservation sont en outre ajoutés en une quantité de 0,01 à 10 % en poids à la composition capillaire régénérante.

3. Utilisation d'une composition selon l'une des revendications 1 et 2 pour la fabrication d'une composition pour usage favorisant la régénération des cheveux et empêchant la perte de cheveux.

4. Un procédé pour la préparation d'une composition capillaire régénérante qui comporte, mélangés les uns avec les autres, un extrait d'herbes orientales de 10 à 40 % en poids, un agent d'ajustement de viscosité de 0,1 à 15 % en poids, un agent tensio-actif de 30 à 80 % en poids, et une huile de 0,1 à 15 % en poids, l'extrait d'herbes orientales étant obtenu en chauffant les herbes orientales avec de l'eau,
où l'extrait d'herbes orientales est obtenu en ajoutant 30 à 70 kilogrammes d'herbes orientales à 800 kilogrammes d'eau, en chauffant d'abord le mélange à 100°C pendant 3 à 8 heures, puis pendant 48 heures ou plus afin d'obtenir un extrait intermédiaire de 600 à 700 kilogrammes, et enfin en chauffant l'extrait intermédiaire afin d'obtenir un extrait final de 400 à 500 kilogrammes,
où les 30 à 70 kilogrammes d'herbes orientales comprennent 5 à 30 kilogrammes de ginseng et 20 à 45 kilogrammes de chrysanthemum zawadskii.

5. Le procédé selon revendication 4, où un ou plusieurs additifs choisis parmi un groupe se composant se composant d'un colorant, d'un arôme, et d'un agent de conservation sont en outre ajoutés en une quantité de 0,01 à 10 % en poids à la composition capillaire régénérante.
